# EUROPEAN PATENT SPECIFICATION

(11) **EP 2 046 815 B1**
(45) Date of publication and mention of the grant of the patent: **08.08.2012**
(21) Application number: 07784915.6
(22) Date of filing: 19.07.2007
(51) Int. Cl.: C07K 7/64, C07K 7/08, C07K 14/46, C07K 14/81

(54) **ANTI-INFLAMMATORY AND ANTIALLERGIC CYCLIC PEPTIDES**
ENTZÜNDUNGSHEMMENDE UND ANTIALLERGISCHE CYCLISCHE PEPTIDE
PEPTIDES CYCLIQUES ANTI-INFLAMMATOIRES ET ANTIALLERGIQUES

(30) Priority: 21.07.2006 BR PI0602885; 04.07.2007 BR PI0703175
(43) Date of publication of application: 15.04.2009
(73) Proprietor: CRISTÁLIA PRODUTOS QUÍMICOS FARMACÊUTICOS LTDA., 13970-000 Itapira (BR); Fundação de Amparo à Pesquisa do Estado de São Paulo - FAPESP, 05468-901 São Paulo (BR); Ferreira, Mônica, 05503-900 São Paulo (BR)
(72) Inventor: FERREIRA, Mônica, 05503-900 São Paulo (BR); SILVA, Carla, 05503-900 São Paulo (BR); PIMENTA, Daniel, 05503-900 São Paulo (BR); PORTARO, Fernanda, 05503-900 São Paulo (BR); CONCEIÇÃO, Kátia, 05503-900 São Paulo (BR); DEMASI, Marilene, 05503-900 São Paulo (BR)
(74) Representative: Alves Moreira, Pedro
(86) International application number: PCT/BR2007/000186
(87) International publication number: WO 2008/009085

(56) References cited:
- WO-A1-03/099862
- US-A1- 2003 166 003
- QI RUI-FENG ET AL: "Structural features and molecular evolution of Bowman-Birk protease inhibitors and their potential application" ACTA BIOCHIMICA ET BIOPHYSICA SINICA, BLACKWELL PUBLISHING, INC., MALDEN, MA, US LNKD- DOI:10.1111/J.1745-7270.2005.00048.X, vol. 37, no. 5, 1 May 2005 (2005-05-01), pages 283-292, XP002524245 ISSN: 1672-9145
- MAGALHAES G.S. ET AL.: 'Natterins, a new class of proteins with kininogenase activity characterized from Thalassophryne nattereri fish venom' BIOCHIMIE vol. 87, no. 8, August 2005, pages 687 - 699, XP004996881

## Description

### Field of the Invention

The present invention refers to synthetic, cyclic peptides containing a sequence of 13 L-amino acids in their primary structure which present anti-inflammatory and antiallergic activities, useful for the treatment of acute or chronic inflammation and/or allergies, being particularly useful for the treatment of acute or chronic allergic asthma. The invention also describes a pharmaceutical composition containing said peptides, its use and a method to treat or prevent acute and/or chronic inflammatory and/or allergic disorders.

### Abbreviations

The following abbreviations will be used throughout the descriptive report:
"Seq01" refers to Sequence ID No. 1;
"Seq02" refers to Sequence ID No. 2;
"Seq03" refers to Sequence ID No. 3;
"Seq04" refers to Sequence ID No. 4;
"Seq05" refers to Sequence ID No. 5;
"Seq06" refers to Sequence ID No. 6;
"Seq07" refers to Sequence ID No. 7;
"Seq08" refers to Sequence ID No. 8;
"Seq09" refers to Sequence ID No. 9.

### Basis of the Invention

Inflammation is a dynamic and protective homeostatic response which occurs as a result of diverse aggressions to which the host is subjected. The acute phase of inflammation is characterized by alterations in the vascular system, which result in an increase in blood flow and structural changes in microcirculation, allowing an efflux of plasmatic proteins and leukocytes from circulation, what is accompanied by a series of events on cell and tissues aiming the homeostasis recovery.

In terms of events in the tissues, in this phase, leukocytes are recruited to the site of injury. Initially, the predominant influx is of neutrophiles, followed by mononuclear cells. This influx is responsible for resolution of the inflammatory process, removing the aggressor agent and restoring tissue homeostasis.

Neutrophiles are the first cells to reach the site of lesion and predominate numerically in the acute phase of tissue injury. They play an important role in defense of the organism by way of phagocytosis and destruction of the causative agent. In addition, neutrophiles may be activated by cytokines produced by resident macrophages in endothelial cells and tissue.

Once blood monocytes leave the circulatory system and enter the site of injury, they differentiate into macrophages, which are the final effector cells in the inflammatory response. The macrophages are responsible for the phagocytosis of foreign particles and destroy them through the synthesis of reactive oxygen species such as nitric oxide. Sequentially, the activated macrophage acts by removing cellular debris, inducing the elimination of the antigen, and the resolution of the inflammatory response, as well as initiating the adaptive immune response.

Considering the cellular events in another way, leukocytes, which were recruited to the site of injury, initiate the process of secretion of pro-inflammatory or "alarm" cytokines (IL-1, TNF-α and IL-6) which initiate a chain of humoral and cellular reactions, at the site of inflammation as well as at distance.

Activation of stroma cells by the cytokines provoke the secretion of chemotactic chemokines which act upon neutrophiles and mononuclear cells. At the inflammation site, vasodilation of the post-capillary venules initially occurs as well as changes in blood flow (a decrease), resulting in margination of the leukocytes along the vascular endothelium, a process mediated by selectins and their ligands, which are rich in carbohydrates.

After activation, the leukocyte stops rolling and firmly adheres to the endothelium. This event is a result of the binding of integrins β1 and β2 expressed in the leukocytes to various members of the immunoglobulins superfamily, expressed in the endothelium (ICAM-1, ICAM-2 and VCAM-1). Finally, the leukocytes migrate between the endothelial cells of the apical region towards the basolateral surface (diapedesis) in the direction of the extravascular space. The subsequent sub-endothelial migration through the extravascular tissue is dependent upon gradients of chemokines, chemotactic cytokines and adhesive interactions with the extracellular matrix. In the end, at the inflammatory focus, the leukocytes increase their cytotoxic functions, liberating oxidizing agents, proteases and other products such as growth factors and cytokines. Eosinophiles, contrary to neutrophiles, can survive in the tissues for long periods, at times for weeks, depending on the cytokines present in the micro-environment.

In relation to the cytokines involved in this process, pro-inflammatory cytokine TNF-α, produced by monocytes, macrophages, activated NK cells and T-lymphocytes, increases expression of adhesive molecules of the endothelial cells; activates neutrophiles; stimulates macrophages to produce IL-1, IL-6, and IL-8; increases expression of class I MHC; increases the synthesis of prostaglandins by hypothalamus cells; acts on hepatocytes in the production of serum amyloid protein; suppresses division of cells in the medulla; reduces tissular perfusion (by diminishing myocardial contractility); relaxes vascular smooth muscle tonus and promotes intra-vascular thrombosis.

Cytokine IL-1, produced by macrophages, endothelial and epithelial cells, promotes the synthesis of IL-2 by T lymphocytes and the differentiation of B lymphocytes; promotes the synthesis of prostaglandins; increases the proliferation of T and B lymphocytes; acts upon macrophages inducing the synthesis of IL-1, IL-6, TNF-α and IL-8; promotes the acute phase synthesis of proteins; cachexia; and increases the division of cells in the medulla.

Cytokine IL-6, produced by macrophages, endothelial and T lymphocytes, acts in hepatocytes in the production of fibrinogen; growth factor for activated B lymphocytes; increases the division of cells in the medulla.

These cellular and tissular events result in effects that are characterized by pain, increase in body temperature, erythema, edema and loss of function near the injury site while inflammation persists. Failures in the regulation of inflammatory process control occur and can lead to epithelial, endothelial or muscle cell destruction as well as result in dysfunction at the cellular level or of the affected organ.

Asthma, for example, is a chronic, pulmonary inflammatory disease characterized by episodes of bronchoconstriction and by eosinophilic pulmonary inflammation. Bronchoconstriction, in the acute phase, is provoked by pharmacological mediators released by mastocytes, activated by the binding of an allergen to allergen-specific IgE molecules on its surface, and the late phase bronchial reactivity is associated with a severe eosinophilic pulmonary inflammation. The T lymphocytes, principally subtype Th2, orchestrate these responses by way of production of cytokines such as IL-4, IL-5, IL-9 and IL-13 which are genetically controlled.

Presentation of the antigen by way of the mature pulmonary dendritic cells is the basis of the process of Th2 sensitization which occurs in allergic patients or in animals exposed to an airborne allergen.

Evidences of inflammation in asthma were initially derived from findings which showed the preferential accumulation of, T CD4⁺ lymphocytes, type Th2, mastocytes and macrophages in the lungs of patients with fatal asthma. Analysis of the bronchoalveolar lavage of asthmatic patients during late phase reactions, LPR, also revealed the presence of an elevated number of T CD4⁺ lymphocytes and eosinophiles.

Activated T CD4⁺ cells are the principal source of cytokines (IL-4, IL-5, IL-9 and IL-13) which are responsible for the development of an allergic pulmonary inflammatory response, typically Th2, with predominance of an eosinophilic infiltrate and production of IgE. The eosinophiles are attracted to the lung due to the action of diverse factors such as cytokines and chemokines which promote chemotaxy, the increase in the expression of adhesive molecules in the vascular endothelium and at the cell surface, activation and cellular transmigration.

Once activated, the eosinophiles release their granular contents (principal basic protein, neurotoxin derived from eosinophiles, cationic protein from eosinophiles and eosinophile generated peroxidase), which, in addition to being toxic to helminthes and bacteria, directly stimulate the smooth bronchial musculature, causing contraction and promoting the increased reactivity towards cholinergic mediators.

In a previously established pulmonary inflammation, eosinophiles are capable of amplifying the response, recruiting new cells, due to the localized production of pro-inflammatory cytokines. Chronic eosinophilic inflammation and IgE antibodies have been directly associated with the severity of the asthma.

Chronic inflammation in asthma is accompanied by pulmonary hyper-reactivity (increased bronchoconstriction in response to non-specific stimuli) and by structural changes in the aerial passages.

Many factors can precipitate an asthma attack, including allergens, bacterial or viral infections, exercise, abrupt meteorological changes, or exposure to airborne irritants, such as tobacco smoke. The principal causes of asthma in children under 5 are viral infections caused by parainfluenza virus (PIV), syncytial respiratory virus (VSR), rhinovirus and enterovirus.

Sensitive asthmatic individuals, when exposed to an allergen in aerosol form, manifest symptoms which begin within 5 to 10 minutes. During this immediate phase of asthma, the following symptoms are observed: contraction of the smooth bronchial musculature, localized edema, increase in the secretion of mucus by the epithelial bronchial glands and a slight cellular infiltration. These characteristics can be explained by the action of mediators such as histamine, prostaglandin D2 (PGD2), leukotriene C4 (LTC4), platelet activating factor (PAF) and cytokines TNF-α and IL-1 released by mastocytes activated by IgE allergen-specific antibodies.

According to estimates by the National Center for Health Statistics of the Center for the Control and Prevention of Diseases (NCHS-CDC, 2005, Hyattsville, MD) approximately 22.2 million people suffer from asthma in the United States, among which 6.5 million are less than 18 years old. In the United States alone, close to 11 people die from asthma per day. In Brazil, estimates show that 10% of the population presents symptoms of asthma, with 2500 deaths per year, being the fourth largest cause for admission to the Universal Health Care System (SUS). Among asthmatic patients, 60% of the cases are difficult to control.

Currently, treatments for inflammation and asthma utilize two categories of drugs: 1) pharmaceutical agents to improve acute symptoms (60% of asthmatics) represented principally by β₂-agonists with a rapid onset of action, ipratropium bromide which is a quaternary ammonium anticholinergic agent and aminophylline, a xanthine derivative which causes relaxation of the smooth muscle of the bronchioles; and 2) maintenance drugs, used to prevent the symptoms of asthma, represented principally by inhaled and systemic corticosteroids.

Nonetheless, use of these drugs, especially repeated or prolonged use, have limitations due to their non-specific action and consequent undesirable side effects for the patients. Furthermore, anti-inflammatory medications currently in use are not able to significantly prevent the loss of pulmonary function associated with asthma.

Drugs which seek to treat inflammation with the potential of minimizing the risk of side effects and which are more specific are being constantly investigated. Several examples may be cited.

Patent application WO 03/070194 presents a proposal to esterify corticosteroids with α-amino acids, thus being able to function as pro-drugs useful for the treatment of rhinitis and asthma, particularly by way of inhalation, and for the treatment of inflammation, particularly by way of local or topical administration. However, the advantage presented by the proposed pro-drugs is not clear as the proposed treatment still depends upon the action of corticosteroids.

Patent application WO 2004/068928 refers to peptides isolated from the defensive secretion from the skin of the toad, *Bombina maxima,* which are agonists of the B₂ bradykinin receptor able to be used to treat and/or prevent disorders associated with bradykinin including cardiovascular disorders, inflammation, asthma, allergic rhinitis, pain, angiogenesis among others.

In yet another patent application, US 2003/0152564, a peptide is presented which corresponds to positions 62-71 of the sequence of human C-reative protein which is able to inhibit, *in vitro,* enzymatic activity of human leucocitary elastase and/or of human cathepsin G, being able to be destined toward the treatment of chronic inflammatory conditions such as rheumatoid arthritis, pulmonary emphysema, cystic fibrosis, bronchitis, asthma and some acute syndromes involving respiratory disturbances.

Patent application US 2007/0123455 proposes a method and compositions which comprise the human proteins S100A8 and/or S100A9, said to be adequate to treat inflammatory disorders such as allergies, asthma, atherosclerosis, autoimmune diseases, infections, among others.

US patent 5,290,762 describes a method for prophylaxis or treatment of inflammatory diseases in a patient which comprises the administration to the location of injury, of a quantity of at least one inhibitor of serine protease. The document claims, as a group of serine protease inhibitors, any inhibitor of the secretion of leucocytic protease, C-reactive protein, serum amyloid protein A, alpha-2-macroglobulin or alpha-2-antiplasmin.

The involvement of the inhibition of serine protease for the treatment of inflammation has been widely investigated. For example, human tryptase is a serine protease, found in the mastocytes, similar to trypsin. The tryptase is the mediator of a series of allergic and inflammatory pathologies, including rhinitis, conjunctivitis and asthma. As such, inhibitors of this serine protease could be utilized with success for the treatment of allergic and respiratory diseases.

Among the inhibitors of serine protease, the best studied are the peptides described by Bowman [Proc. Soc. Expd. Med. 1946 63:547] and Birk et. al. [Bull. Res. Council Israel, Sec A 1962 11:48; Biochem. Biophys. Acta 1963 67:326] (which are the origin of the denomination BBI - *Bowman Birk* Inhibitors - originated). BBI's are encountered abundantly in dicotyledonous and monocotyledonous plants. Among these, the named SFTI-1 (*sunflower trypsin inhibitor-1*) is pointed out, which is a bicyclic peptide composed of 14 amino acids, up to the present, is the smallest and most potent natural Bowman-Birk type inhibitor. SFTI-1 has been used in transgenic plants against pathogens and insects, but may also be utilized in the prevention of cancer, dengue fever and other inflammatory and allergic diseases.

[QI Rui Feng et al., Structural Features and Molecular Evolution of Bowman-Birk protease inhibitors and their potential application, Acta Biochimica et Biophysica Sinica, 2005, 37(5): 283-292].

In addition to pharmacologically active peptides can be produced by plants, substances produced by animals, for example, venoms, have also been the targets of investigation. Animal toxins may be considered to be molecules developed by nature capable of reaching specific and selective targets, inhibiting or stimulating physiological reactions.

Particularly, the proteins denominated natterines, which are isolated from the venom of the fish Thalassophryne nattereri found in Brazil, should be pointed out. These proteins form a family of toxins with a molecular mass around 38 kDa, whose sequences present a high degree of homology among them and are capable of inducing innumerous biological activities such as edema and nociception.

Using the natterines as a starting point, it could be observed sequences of amino acids which are not obtained per se by way of purification of the natural venom, although stand out with respect to their structural similarity to the Bowman-Birk peptides. Attempts to isolate said sequences of amino acids from the natterines are frustrated, since it requires a long and laborious process, resulting in very small quantities of impure peptide fragments, not exactly correspondent to the peptides of interest of the present invention. This is a significant impediment to the industrial production of these peptides.

### Summary of the Invention

The present invention presents cyclic peptides as defined in claim 1 They can have a sequence of 13 L-amino acids in their primary structure, presenting specifically the amino acid cysteine in positions 4 and 13 of the peptide chain and the amino acid lysine in position 11 of the peptide chain. They are characterized by containing a disulfide bridge formed between the thiol groups of the 4 and 13 cysteine residues, forming a cyclic peptide. Said synthetic, cyclic peptides present anti-inflammatory and antiallergic activity, particularly with potential application in the treatment of acute and/or chronic asthma.

### Description of Figures

**Figure 1****:** Michaelis-Menten kinetics obtained for the hydrolysis of the substrate Abz-FRSSRQ-EDDnp by the catalytic activity of trypsin.
**Figure 2****:** Effect of the different types of Seqs on leucocyte rolling induced in the microcirculation by LPS (lipopolysaccharide). * *p* < 0.05 compared with the LPS group.
**Figure 3****:** Effect of the change in essential amino acids in the sequence of Seq07 and Mod_{Lys/Ala} on the rolling of leukocytes induced in the microcirculation by LPS (lipopolysaccharide). * *p* < 0.05 compared with the LPS group.
**Figure 4****:** Prevention of LPS (lipopolysaccharide) induced peritonitis by Seq07. Mice injected with LPS (10 µg/mL) were treated intraperitoneally 30 minutes **before** with Seq07 (4 or 40 nM). The figure represents the total number of cells (***A***), neutrophiles (***B***) and macrophages (***C***) of the peritoneal washing after 24 hours. The results are expressed as the median ± SEM. * *p* < 0.05 compared to the LPS group.
**Figure 5****:** Effect of Seq07 (4 or 40 nmol) on the recruitment of leukocytes and eosinophiles in the brochoalveolar lavage in acute asthma. The bronchoalveolar lavage of mice was submitted for counting of the total number of cells (***A***) and eosinophiles (***B***). The results represent the median ± SEM of 6 animals/group. * *p* < 0.001 compared to the Asthma group.
**Figuro 6:** Effect of Seq07 on the recruitment of leukocytes and eosinophiles in the bronchoalveolar lavage in chronic asthma. The bronchoalveolar lavage was submitted for counting of the total number of cells (***A***) and eosinophiles (***B***). The results represent the median ± SEM of 6 animals/group. * *p* < 0.001 compared to the Asthma group.

### Detailed Description of the Invention

The present invention refers to cyclic, synthetic peptides as defined in claim 1 presenting, preferably, chemical protecting groups at one or both of the extremities of the peptide, said peptides presenting anti-inflammatory and antiallergic activities.

The peptides of the present invention possess the amino acid cysteine at the 4 and 13 positions of the peptide chain and the amino acid lysine at the 11 position of the peptide chain.

Additionally, the peptides included in the present invention preferably possess the amino acids valine, leucine or isoleucine or another hydrophobic and neutral amino acid at position 1 of their basic sequence.

Additionally, the peptides included in the present invention preferably possess the amino acids arginine, glutamine or another hydrophilic and polar amino acid, capable of possessing a positive charge, at position 3 of their basic sequence.

Additionally, the peptides included in the present invention preferably possess the amino acids arginine, threonine, serine, aspartate or another hydrophilic and polar amino acid at position 5 of their basic sequence.

Additionally, the peptides included in the present invention preferably possess the amino acids methionine, isoleucine, leucine or another hydrophilic and neutral amino acid at position 7 of their basic sequence.

Additionally, the peptides included in the present invention preferably possess the amino acids glycine or aspartate at position 9 of their basic sequence.

Particularly, the peptides of the present invention are characterized by the fact that they contain a disulfide bridge formed between the thiol groups of the 4 and 13 cysteine residues, forming a cyclic peptide.

Chemical protection of the peptide molecule may be accomplished by the addition of chemical protecting groups to the carboxy terminal extremity of the peptide, or at the amino terminal extremity of the peptide, or yet still by the protection of both the extremities of the molecule. Preferably, protection of the molecule occurs by way of amidation of the carboxyl group of the amino acid cysteine at position 13 of the peptides of the present invention, or by way of acetylation of the amino acid residue at position 1 of the peptide chain, or still, by protection of both the carboxy terminal and the amino terminal extremities.

In the main embodiment of the present invention, the peptides are characterized by the sequences of amino acids identified in this document by way of the abbreviations Seq01, Seq02, Seq03, Seq04, Seq05, Seq06, Seq07, Seq08 and Seq09.

Also included within the scope of the present invention are the individual, alternate or combined substitution of proline residues by hydroxylated proline (hydroxyproline); the individual, alternate or combined substitution of methionine residues by an oxidized form of methionine; and the individual, alternate or combined substitution of L-amino acids by D-amino acids.

The cyclic peptides included in the present invention, alone, or in a combination containing at least two of the peptides, present the capacity to inhibit the adhesion and rolling of leukocytes in mammals tested in the laboratory. In addition to the inhibition of adhesion and rolling of the leukocytes, there is also observed a decrease in, and a prevention of the recruiting of leukocytes, particularly neutrophiles and macrophages, in the peritoneal cavity of laboratory mammals injected with the inflammatory agent LPS (lipopolysaccharide), when treated with representative peptides of the present invention.

In immunized mammals challenged with ovalbumin (OVA), which causes the manifestation of acute murine asthma, the cyclic peptides of the present invention, alone or in a combination containing at least two of the peptides, when administrated intramuscularly, intranasally and orally, have also been shown capable of inhibiting the recruitment of the total number of leukocytes toward the alveolar space, this decrease being characterized particularly by the absence of eosinophiles in the bronchoalveolar lavage.

The cyclic peptides of the present invention, alone or in a combination containing at least two of the peptides, are also able to reduce the total number of leukocytes recruited to the alveolar space, with a reduction in eosinophiles, in a conventional experimental model for the development of chronic asthma in animals.

In light of the characteristics heretofore related, it has been observed that the cyclic peptides of the present invention, alone or in a combination containing at least two of the peptides, are capable of inhibiting, in mammals, inflammatory manifestations, pathological allergic manifestations where an influx of eosinophiles is observed such as asthma, bronchitis, rhinitis and dermatitis, in addition to associated pathological manifestations such as arthritis, disturbances of the immune system, lymphocytic dysfunction during the immune response, tumors, cellular adhesion and/or manifestations of parasitic diseases. The results obtained for asthma were compared with the conventional treatment for asthma, utilizing dexamethasone, in which the peptides of the present invention were shown to be equally efficient.

Additionally, it has been observed that the cyclic peptides of the present invention are resistant to digestion with trypsin, thus being able to be administered orally. Other means of administration which may be employed for the peptides of the present invention include, but are not limited to, intranasal, intramuscular and intravenous administration.

In addition to being resistant to the action of the enzyme trypsin, the peptides of the present invention are also able to inhibit the proteolytic action of trypsin, presenting a Kᵢ in the range of 20 to 63.6 nM. This property of the peptides of the present invention suggests that such peptides may be classified as inhibitors of serine protease, once trypsin is an important enzyme representative of the family of serine proteases. Knowing also that human tryptase is a serine protease found in the mastocytes, being very similar to trypsin, and that its inhibition has shown to be successful in the treatment of respiratory and allergic diseases, it can be suggested that the activity verified for the peptides of the present invention is related to the inhibition of serine proteases.

The peptides of the present invention do not possess any cytotoxic effect on macrophages, and also do not demonstrate any damaging effect on muscular fibers or on microcirculation in the cremaster muscle of laboratory animals.

Furthermore, the peptides of the present invention, alone or in combination, have not been shown to be immunogenic, in that they do not induce the synthesis of specific antibodies and, above all, have not been shown to be lethal.

In one embodiment of the present invention, the cyclic peptides Seq01, Seq02, Seq03, Seq04, Seq05, Seq06, Seq07, Seq08 and Seq09 may be obtained employing known techniques for the synthesis of peptides, such as classical chemical synthesis in solution or in the solid phase; enzymatic synthesis; or by way of techniques using recombinant DNA.

The chemical synthesis of the cyclic peptides of the present invention may be preferably accomplished in the solid phase, and more preferably, with use of the FMOC (9-Fluorenylmethoxycarbonyl) strategy to protect the alpha-amino groups present in the reaction.

The purification of the synthetic peptides may be accomplished employing chromatographic techniques, well known in the state of the art. Preferably, liquid chromatography is employed, the purity and identity of the peptides being confirmed by mass spectrometry.

After purification of the synthesized peptides according to the above description, it is then necessary to introduce the disulfide bridges into the respective peptides. The disulfide bridges are formed between cysteine residues at positions 4 and 13 in the same peptide chain by the method of reduction in air, for example, by dissolving the peptide in a solution of 0.01 to 0.5M ammonium bicarbonate in a proportion of 0.05 to 15 mg/mL with agitation until complete formation of the disulfide bridge. The peptides may be isolated by lyophilization and purified by way of preparative liquid chromatography.

Another embodiment of present invention refers to a pharmaceutical composition containing the peptides Seq01, Seq02, Seq03, Seq04, Seq05, Seq06, Seq07, Seq08 or Seq09, which may be in the form of pharmaceutically acceptable salts of the same, alone or in a combination containing at least two of the peptides, in one or more vehicles chosen among a diluent, an excipient or a pharmaceutically acceptable solvent.

The present invention discloses a method to treat or prevent inflammatory and/or allergic disorders, acute or chronic, such as, but not limited to, bronchial asthma, rhinitis, arthritis, atopic dermatitis, disturbances of the immune system, lymphocytic dysfunction during the immune response, tumors, cellular adhesion and/or manifestations of parasitic diseases, by way of administration, to a mammal, the cyclic peptides Seq01, Seq02, Seq03, Seq04, Seq05, Seq06, Seq07, Seq08 or Seq09, alone or in combination, or of pharmaceutical compositions containing at least one of the peptides of the present invention.

Yet another embodiment of the present invention is the use of the cyclic peptides Seq01, Seq02, Seq03, Seq04, Seq05, Seq06, Seq07, Seq08 and Seq09, alone or in a combination containing at least two of the peptides, in the manufacture of medicines employed in the prevention or treatment of inflammatory and/or allergic disorders, acute or chronic, such as, but not limited to, bronchial asthma, rhinitis, arthritis, atopic dermatitis, disturbances of the immune system, lymphocytic dysfunction during the immune response, tumors, cellular adhesion and/or manifestations of parasitic diseases. According to this aspect, the synthetic, cyclic peptides of the present invention, alone or in combination, may be used to obtain a pro-drug or drug which inhibits the development of inflammatory and/or allergic conditions in mammals.

The administration of the cyclic peptides of the present invention or of pharmaceutical compositions containing the same, may be accomplished by way of, but not limited to, intramuscular, intranasal, intravenous and oral administration, the preferred method of administration being orally.

The following examples are cited to illustrate the embodiments of the present invention and must be employed only for a better comprehension of the developments contained at the present invention but should not be construed as to limit the scope or spirit of the invention.

### EXAMPLE 1A: Inhibition of Trypsin

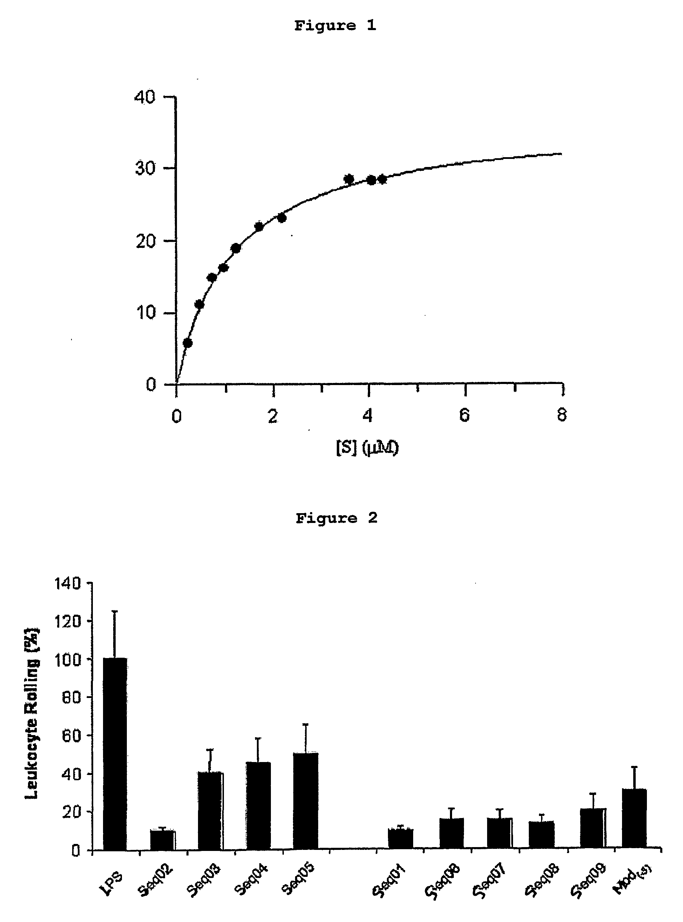

Studies on the inhibition of trypsin with peptide Seq01 revealed a simple, competitive inhibition, as shown in Figure 1 (simple intercept with the X axis) and yielded a value of Kᵢ in the range of 10µM - 0.1 nM.

The substrate utilized was Abz-Phe-Arg-Ser-Ser-Arg-Gln-EDDnp, a fluorescent analog of bradykinin, purified by way of high pressure liquid chromatography, (HPLC) until purity greater than 95% (single peak) was achieved.

### EXAMPLE 1B: Tests:

### a) Enzymatic reactions for the determination of Kₘ and k_{cat}

The concentration of the substrate solution was determined by fluorometric analysis of the product Abz formed after complete hydrolysis by trypsin.

The hydrolysis reactions were conducted using a Hitachi F-2000 spectrofluorometer, with the excitation and emission slits set at 10 and 20 nm, and the excitation and emission wavelengths at 320 and 420 nm, respectively. The temperature of the reaction cuvette was maintained at 37°C in a thermostatically controlled compartment.

The enzymatic activity of trypsin (0.5 - 5 nM) was determined in 50 mM Tris-HCl buffer, pH 8.0, containing 20 mM NaCl at 37°C, with the addition of approximately 0.1 - 6.0 µM of the fluorescent substrates. The increase in fluorescence was monitored continually for 5 min in the spectrofluorometer. When necessary, the correction of the fluorescent reading was done using the empiric equation described by Araújo et. al., Biochemistry 25; 8519, 2000. Kinetic data were determined from the initial hydrolysis (consumption of substrate less than 10%) and calculations involving kinetic parameters (Kₘ, Vₘₐₓ and k_{cat}) were performed using the Michaelis-Menten equation furnished by the GRAFIT for Windows 3.0 program.

### b) Determination of the inhibition constants (Kᵢ)

To determine the inhibition kinetics of trypsin with the peptides of the present invention, three substrate concentrations were used: a) corresponding to half of the value of Kₘ (0.8 µM); b) corresponding to the whole value of Kₘ (1.6 µM); c) corresponding to slightly more than double the value of Kₘ (3.5 µM). The enzyme was employed at a concentration of 3.0 nM and the inhibitors (Seq01 to Seq09) at three concentrations (10 nM, 100 nM and 1µM), in addition to a control for each substrate concentration, the substrate consumption being less than 10% of the initial concentration. Incubation carried out at 37°C Tris-HCl buffer, pH 8.0, containing 20 mM NaCl at 37°C. For characterization of enzymatic inhibition, a Lineweaver-Burk plot was employed (1/V vs 1/S), and the value of Kᵢ was determined from the relation between the apparent inhibition constant (Ki₍ₐₚₚ₎) and the value of Kₘ for the substrate: Ki₍ₐₚₚ₎ = [I]/[(v₀/vᵢ-1] and Ki = Ki(ₐₚₚ)/[(Kₘ/[S])+1] where; [I] = inhibitor concentration; (v₀/v₁) = ratio of the hydrolysis velocities in the absence and presence of inhibitor, respectively. Kₘ/[S] = ratio between Kₘ of the substrate and substrate concentration.

The results obtained are summarized in table 1 below:

**Table 1**

| **Identifying Sequence** | **Ki (nM)** |
|---|---|
| Seq01 | 22.5 |
| Seq02 | 24.0 |
| Seq03 | 63.6 |
| Seq04 | 42.0 |
| Seq05 | 44.0 |
| Seq06 | 24.2 |
| Seq07 | 20.0 |
| Seq08 | 24.8 |
| Seq09 | 21.8 |

### EXAMPLE 2: Inhibition of adhesion and rolling of Leukocytes in vivo :

### a) Rolling of Leukocytes for the peptides of the present invention:

The test to verify the inhibition of adhesion and rolling of leukocytes was performed in mice (n = 3/per group), which were anaesthetized with sodium pentobarbital (Hypnol^{®}, 50 mg/Kg) and maintained under a temperature controlled plate (37°C). Surgical manipulation of the scrotal sac was performed to expose the cremaster muscle, which was fixed around a transparent area of the plate. This was positioned over the chariot of the optical microscope to permit *in vivo* visualization of the local microcirculation. The preparations were maintained humid and warmed by irrigation with 0.15 M PBS (phosphate buffer solution). The inflammatory agent LPS (lipopolysaccharide), at a concentration of 1 µg/mL, diluted to 20 µL, was topically applied to the cremaster muscle. The aspect of the post-capillary venule was registered before and after the application of the LPS (lipopolysaccharide). The peptides Seq01 to Seq09 (1 µM) were applied topically 15 min after application of the LPS. The pre and post-capillary venules, arterioles and muscular fibers events were observed for 30 minutes. The rolling of the leukocytes, the firm adherence and the leakage at the post-capillary venules between 20-40 µm in diameter were registered and quantified in 1 minute intervals for a period of 10 minutes. Transmigration was defined as the number of leukocytes in the extravascular tissue over a segment extending 100 µm. Microcirculation of the cremaster was analyzed by the epi-illumination technique using an intra-vital fluorescence microscope (Axio Imager A1, Carl Zeiss, Germany) as described by Sperandio et. al. (J. Exp. Med., 19; 197(10): 1355-1363, 2003).

The peptides tested induced a decrease in the rolling of the leukocytes in the post-capillary venules of the cremaster muscle of mice on the order of 50 to 90%, as can be seen in Figure 2.

### b) Rolling of Leukocytes with a modified peptide: Deletion of the amino acids at positions 1, 2 and 3 (for illustrating only)

The test above was repeated with a modified peptide (Mod₋₃) from which the amino acids at positions 1, 2 and 3 were removed as represented below:

| | |
|---|---|
| Seq01 | |
| Mod₍₋₃₎ | |

This modification had as objective to verify the need of the cyclic structure resulting from the disulfide bridge between the thiol groups present in the cysteine residues at positions 4 and 13 for anti-inflammatory activity of the peptides of the present invention. The peptide from which the amino acids had been removed maintained inhibitory activity with respect to the rolling of leukocytes in comparison to the peptides described in the present invention, as can be seen in Figure 2.

### c) Effect of the substitution of Lys at position 11 on the activity of the peptides of the present invention

The test for leucocyte rolling was repeated, as described in example 2, for a modified peptide (Mod_{Lys/Ala}) in which lysine at postion 11 of one of the peptides of the present invention was substituted by alanine (an amino acid with properties contrary to lysine) to determine the essentiality of the Lys₁₁ residue for the activity of the peptides of the present invention as is represented below:

| | |
|---|---|
| Seq01 | |
| Mod_{Lys/Ala} | |

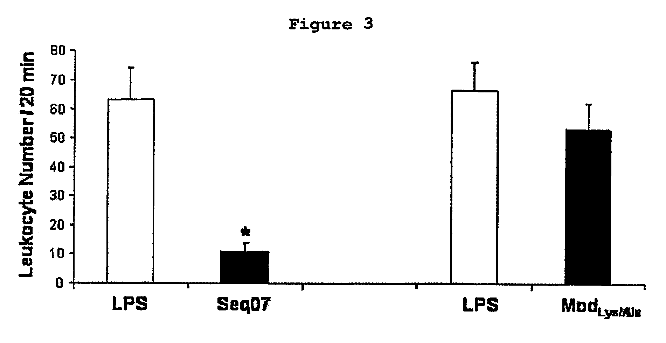

The modified peptide, (Mod_{Lys/Ala}), was not efficient in the inhibition of leucocyte rolling when compared to peptide Seq07, as can be seen in figure 3, which demonstrates the importance of the presence of Lys₁₁ to maintain pharmacological activity.

### EXAMPLE 3: Prevention of peritonitis induced by LPS

Mice were injected intraperitoneally with 4 nmol or 40 nmol of the peptide identified by Seq07 dissolved in 500 µL of sterile saline solution, 30 minutes prior to administering intraperitoneal injection of LPS (O55:BR 20 µg/mL, Sigma) diluted in 500 µL of sterile saline solution. Animals injected only with sterile saline were considered controls. 24h after injection of LPS, the animals were sacrificed with a high dose of chloral hydrate to obtain the washing of the peritoneal cavity to count the number of cells (A), neutrophiles (B) and macrophages (C). The material removed from the peritoneum was centrifuged for 10 minutes at 1500 rpm and 4°C, the supernatant was separated and frozen at -20°C for future analyses, while the remaining material containing the cells was re-suspended in PBS 0.1% BSA. The total count was performed in a Neubauer chamber. For the differential count, an aliquot of the cellular suspension was placed on slides and centrifuged (Citospin), stained with Hema 3 and examined under the optical microscope, counting a total of 300 cells. The absolute number of each cellular population was obtained by multiplication of the percentages by the total number of cells found in the sample volume.

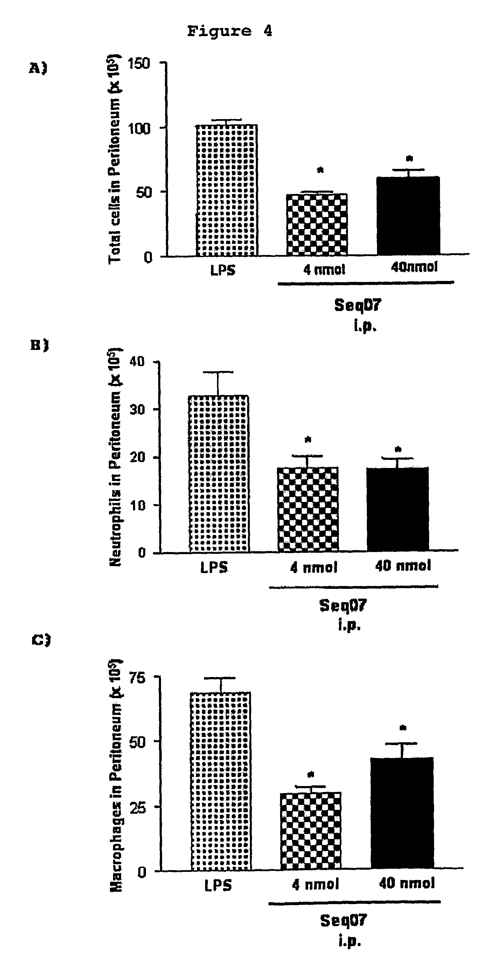

Both concentrations of the peptide tested (4 nmol and 40 nmol) prevented close to 50% of the recruitment of leukocytes in the peritoneal cavity of mice injected with LPS (compared to the control). It can also be verified by examining Figure 4, that the peptide prevented the recruitment of neutrophiles and macrophages.

### EXAMPLE 4: Inhibition of the Acute and Chronic Pulmonary Allergic and Inflammatory Response

Induction of acute pulmonary asthma was observed in BALB/c mice, 7 week old, males, which were immunized with 1% ovalbumin (OVA grade V, 10 µg, Sigma) adsorbed on 1.6 mg of aluminum hydroxide on days 0 and 7. After the 14^{th} day, the animals were submitted to 3 challenges per week with a 1% OVA aerosol. Immunized animals and challenged only with PBS (phosphate buffered saline) were considered controls. To induce chronic asthma, the animals were challenged 3 times a week during 3 consecutive weeks.

For the aerosol challenge, the animals were placed in a closed plastic box adapted with an ultra-sonic nebulizer (US-800, ICEL) and exposed to inhalation of 2 mL of a 1% solution of ovalbumin (grade V, Sigma) in saline, over 20 minutes. 24h after the last aerosol challenge, the animals were sacrificed with a high dose of chloral hydrate to obtain the peripheral blood, the Broncho-Alveolar Lavage (BAL) and the pulmonary tissue to count the total number of leukocytes and eosinophiles.

Groups of animals were treated with 4 nmol or 40 nmol of the peptide identified by Seq07 intranasally and intraperitoneally 30 minutes before each challenge, and orally 1 hour before each challenge. The group of animals with chronic asthma was treated only with 4 nmol or 40 nmol of the peptide identified by Seq07 intraperitoneally 30 minutes before each challenge.

To obtain the BAL, the animals of both groups (OVA treated or Control) were sacrificed with 10% chloral hydrate, the blood removed, and had a cannula placed in the trachea. The aerial space was washed with three, 1 mL aliquots of HBSS *(Hanks Balanced Salt Solution)* + EDTA (*ethylenediaminetetraacetic acid*). After collection, the BAL was immediately centrifuged at 800 rpm for 10 minutes, the supernatant discarded, and the cellular button was re-suspended in 1 mL of HBSS + 0.1% BSA (*bovine serum albumin*) to count the cells. The total cells from the BAL were counted in a Neubauer chamber. Aliquots containing 5 x 10⁵ cells were centrifuged on glass slides using a cytocentrifuge, for 5 minutes at 600 rpm. To determine the percentages of the different cellular populations, the slides were stained with Hema 3 and 300 cells were counted in randomly selected fields. The absolute number of each cellular population in the BAL was obtained by multiplication of the percentages by the total number of cells found in the sample volume.

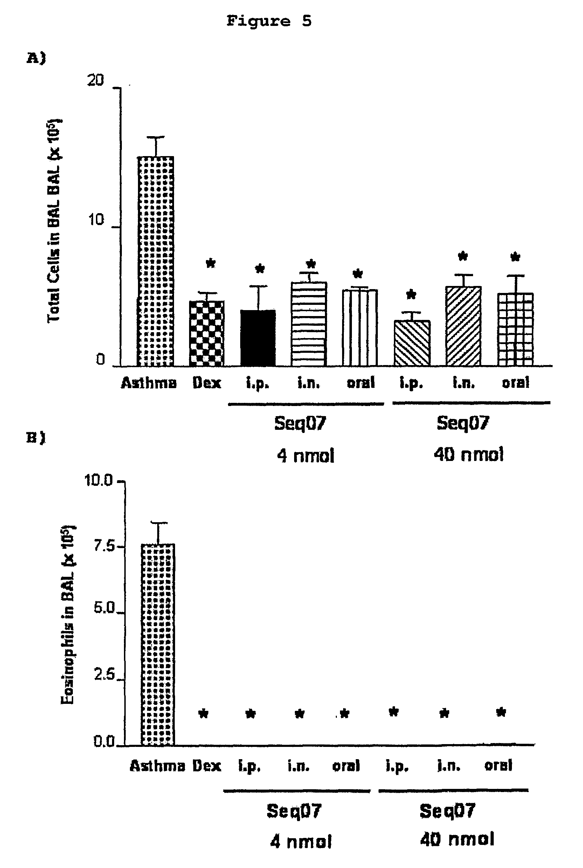

Treatment of animals presenting acute asthma, with 4 nmol or 40 nmol of the peptide tested, by all route of administration, provoked a decrease in the total number of leukocytes recruited to the alveolar space. This decrease was characterized by the absence of eosinophiles, as can be verified in Figure 5. The effect of the peptide tested on the recruitment of cells to the lung was similar to the effect induced by oral dexamethasone at a dose of 0.3 mg/Kg.

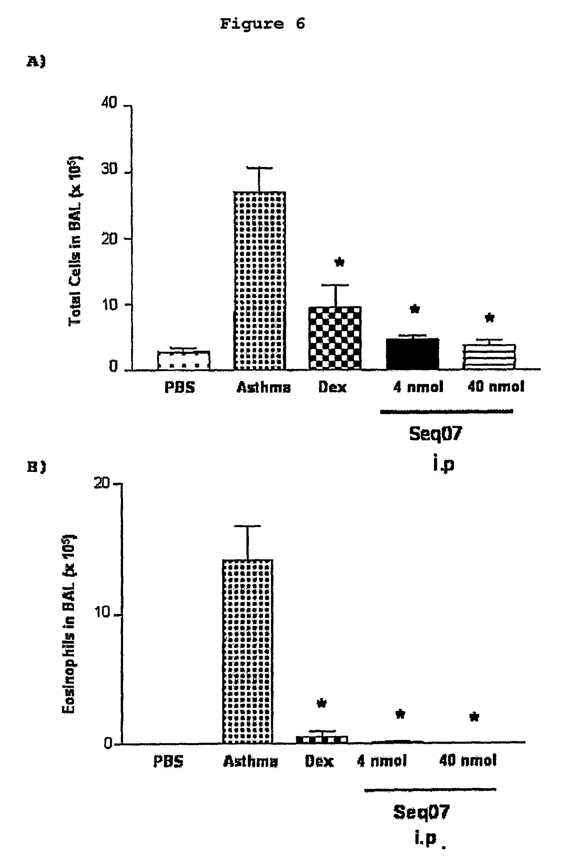

Intraperitoneal treatment of animals presenting chronic asthma, with 4 nmol or 40 nmol of the peptide tested, provoked a decrease in the total number of leukocytes recruited to the alveolar space, with a decrease in eosinophiles, as can be verified in Figure 6. The effect of the peptide tested on recruitment of cells to the lung was similar to the effect induced by oral dexamethasone at a dose of 0.3 mg/Kg.

## Claims

1. A Synthetic, cyclic peptide charactherized by consisting of 13 amino acids, presenting the amino acid cysteine at positions 4 and 13 and the amino acid lysine at position 11 of the peptide chain and a disulfide bridge between the thiol groups of cysteine residues 4 and 13 which possess ant-inflammatory and/or anti-allergic activity.

2. The peptide according to claim 1 **characterized by** having an amino acid sequence selected from the group consisting of:
Ile₁-Pro₂-Arg₃-Cys₄-Arg₅-Lys₆-Met₇-Pro₈-Gly₉-Val₁₀-Lys₁₁-Met₁₂-Cys₁₃ (Seq. ID No. 1);
Val₁-Glu₂-Gln₃-Cys₄-Thr₅-Ile₆-Ile₇-Gly₈-Asp₉-Glu₁₀-Lys₁₁-Asp₁₂-Cys₁₃ (Seq. ID No. 2);
Val₁-Glu₂-Gln₃-Cys₄-Thr₅-Tle₆-Ile₇-Gly₈-Asp₉-Ala₁₀-Lys₁₁-Asp₁₂-Cys₁₃ (Seq. ID No. 3);
Val₁-Gln₂-Gln₃-Cys₄-Ser₅-Glu₆-Ile₇-Ala₈-Gly₉-Ala₁₀-Lys₁₁-Pro₁₂-Cys₁₃ (Seq. ID No. 4);
Leu₁-His₂-Arg₃-Cys₄-Asp₅-Lys₆-Ile₇-Ala₈-Asp₉-Ala₁₀-Lys₁₁-Pro₁₂-Cys₁₃ (Seq. ID No. 5);
Ile₁-Pro₂-Arg₃-Cys₄-Arg₅-Ala₆-Met₇-Pro₈-Gly₉-Val₁₀-Lys₁₁-Met₁₂-Cys₁₃ (Seq. ID No. 6);
Ile₁-Pro₂-Arg₃-Cys₄-Arg₅-Lys₆-Met₇-Pro₈-Gly₉-Val₁₀-Lys₁₁-Met₁₂-Cys₁₃-NH₂ (Seq. ID No. 7);
Ac-Ile₁-Pro₂-Arg₃-Cys₄-Arg₅-Lys₆-Met₇-Pro₈-Gly₉-Val₁₀-Lys₁₁-Met₁₂-Cys₁₃ (Seq. ID No. 8); and
Ac-Ile₁-Pro₂-Arg₃-Cys₄-Arg₅-Lys₆-Met₇-Pro₈-Gly₉-Val₁₀-Lys₁₁-Met₁₂-Cys₁₃-NH₂ (Seq. ID No. 9).

3. The peptide according to claims 1 or 2 **characterized by** the fact that the amino acids are L-amino acids.

4. The peptide according to claim 2 **characterized by** the fact that the proline residues are individually, alternately or in conjunction substituted by hydroxyproline residues.

5. The peptide according to claim 2 **characterized by** the fact that the methionine residues are individually, alternately or in conjunction substituted by residues of oxidized methionine.

6. The peptide according to claim 2, **characterized by** the fact that the L-amino acids are individually, alternately or in conjunction substituted by D-amino acids.

7. The peptide according to claims 1 or 2, **characterized by** being anti-asthmatic.

8. The peptide according to claim 1, **characterized by** being inhibitors of trypsin.

9. A pharmaceutical, composition **characterized by** comprising a peptide of claims 1 or 2, alone or in a combination containing at least two of the peptides, or their pharmaceutically acceptable salts, and one or more pharmaceutically acceptable excipient, wherein the peptide is present in a dose effective to treat or prevent acute and/or chronic inflammatory and/or allergic disorders.

10. The pharmaceutical composition according to claim 9, **characterized by** being administered orally, intranasally, intramuscularly or intravenously.

11. Use of at least 1 of the peptides as described in claims 1 or 2, **characterized by** being employed in the manufacture of a medicine for the treatment or prevention, in a mammal, of acute and/or chronic inflammatory and/or allergic diseases, asthma, rhinitis, arthritis, atopic dermatitis, immune system disturbances, lymphocytic dysfunction during an immune response, tumors, cellular adhesion and/or parasitic diseases.

12. Use according to claim 11, **characterized by** the fact that the peptides .are administered orally, intranasally, intramuscularly or intravenously.

13. Peptides, according to any of claims 1 to 8 for use in the treatment of acute and/or chronic inflammatory and/or allergic diseases, asthma, rhinitis, arthritis, atopic dermatitis, immune system disturbances, lymphocytic dysfunction during an immune response, tumours, cellular adhesion and/or parasitic diseases.

## Patentansprüche

1. Synthetisches, cyclisches Peptid, **dadurch gekennzeichnet, dass** es aus 13 Aminosäuren besteht, die Aminosäure Cystein an den Positionen 4 und 13 und die Aminosäure Lysin an Position 11 der Peptidkette und eine Disulfidbrücke zwischen den Thiolgruppen der Cysteinreste 4 und 13 aufweist, mit entzündungshemmender und/oder antiallergischer Wirkung.

2. Peptid nach Anspruch 1, **dadurch gekennzeichnet, dass** es eine Aminosäuresequenz aus der Gruppe bestehend aus:
Ile₁-Pro₂-Arg₃-Cys₄-Arg₅-Lys₆-Met₇-Pro₈-Gly₉-Val₁₀-Lys₁₁-Met₁₂-Cys₁₃ (Seq. ID Nr. 1);
Val₁-Glu₂-Gln₃-Cys₄-Thr₄-Ile₆-Ile₇-Gly₈-Asp₉-Glu₁₀-Lys₁₁-Asp₁₂-Cys₁₃ (Seq. ID Nr. 2);
Val₁-Glu₂-Gln₃-Cys₄-Thr₅-Ile₆-Ile₇-Gly₈-Asp₉-Ala₁₀-Lys₁₁-Asp₁₂-Cys₁₃ (Seq. ID Nr. 3);
Val₁-Gln₂-Gln₃-Cys₄-Ser₅-Glu₆-Ile₇-Ala₈-Gly₉-Ala₁₀-Lys₁₁-Pro₁₂-Cys₁₃ (Seq. ID Nr. 4);
Leu₁-His₂-Arg₃-Cys₄-Asp₅-Lys₆-Ile₇-Ala₈-Asp₉-Ala₁₀-Lys₁₁-Pro₁₂-Cys₁₃ (Seq. ID Nr. 5);
Ile₁-Pro₂-Arg₃-Cys₄-Arg₅-Ala₆-Met₇-Pro₈-Gly₉-Val₁₀-Lys₁₁-Met₁₂-Cys₁₃ (Seq. ID Nr. 6);
Ile₁-Pro₂-Arg₃-Cys₄-Arg₅-Lys₆-Met₇-Pro₈-Gly₉-Val₁₀-Lys₁₁-Met₁₂-Cys₁₃-NH₂ (Seq. ID Nr. 7);
Ac-Ile₁-Pro₂-Arg₃-Cys₄-Arg₅-Lys₆-Met₇-Pro₈-Gly₉-Val₁₀-Lys₁₁-Met₁₂-Cys₁₃ (Seq. ID Nr. 8) und
Ac-Ile₁-Pro₂-Arg₃-Cys₄-Arg₅-Lys₆-Met₇-Pro₈-Gly₉-Val₁₀-Lys₁₁-Met₁₂-Cys₁₃-NH₂ (Seq. ID Nr. 9)
aufweist.

3. Peptid nach Anspruch 1 oder 2, **dadurch gekennzeichnet, dass** es sich bei den Aminosäuren um L-Aminosäuren handelt.

4. Peptid nach Anspruch 2, **dadurch gekennzeichnet, dass** die Prolinreste individuell, alternierend oder zusammen durch Hydroxyprolinreste ersetzt sind.

5. Peptid nach Anspruch 2, **dadurch gekennzeichnet, dass** die Methioninreste individuell, alternierend oder zusammen durch oxidierte Methioninreste ersetzt sind.

6. Peptid nach Anspruch 2, **dadurch gekennzeichnet, dass** die L-Aminosäuren individuell, alternierend oder zusammen durch D-Aminosäuren ersetzt sind.

7. Peptid nach Anspruch 1 oder 2, **dadurch gekennzeichnet, dass** sie antiasthmatisch sind.

8. Peptid nach Anspruch 1, **dadurch gekennzeichnet, dass** es sich um Inhibitoren von Trypsin handelt.

9. Pharmazeutische Zusammensetzung, **dadurch gekennzeichnet, dass** sie ein Peptid gemäß Anspruch 1 oder 2 allein oder in einer Kombination, die mindestens zwei der Peptide oder pharmazeutisch unbedenkliche Salze davon enthält, und einen oder mehrere pharmazeutisch unbedenkliche Hilfsstoffe umfasst, worin das Peptid in einer zur Behandlung oder Prävention von akuten und/oder chronischen entzündlichen und/oder allergischen Störungen wirksamen Menge vorliegt.

10. Pharmazeutische Zusammensetzung nach Anspruch 9, **dadurch gekennzeichnet, dass** sie oral, intranasal, intramuskulär oder intravenös verabreicht wird.

11. Verwendung von mindestens 1 der Peptide gemäß Anspruch 1 oder 2, **dadurch gekennzeichnet, dass** es bzw. sie bei der Herstellung einer Medizin zur Behandlung oder Prävention von akuten und/oder chronischen entzündlichen und/oder allergischen Störungen, Asthma, Rhinitis, Arthritis, atopischer Dermatitis, Störungen des Immunsystems, Lymphozytendysfunktion während einer Immunantwort, Tumoren, Zelladhäsion und/oder parasitischen Erkrankungen bei einem Säugetier eingesetzt wird bzw. werden.

12. Verwendung nach Anspruch 11, **dadurch gekennzeichnet, dass** die Peptide oral, intranasal, intramuskulär oder intravenös verabreicht werden.

13. Peptide nach einem der Ansprüche 1 bis 8 zur Verwendung bei der Behandlung von akuten und/oder chronischen entzündlichen und/oder allergischen Störungen, Asthma, Rhinitis, Arthritis, atopischer Dermatitis, Störungen des Immunsystems, Lymphozytendysfunktion während einer Immunantwort, Tumoren, Zelladhäsion und/oder parasitischen Erkrankungen.

## Revendications

1. Peptide cyclique synthétique, **caractérisé en ce qu'**il consiste en 13 acides aminés, présentant l'acide aminé cystéine sur les positions 4 et 13 et l'acide aminé lysine sur la position 11 de la chaîne peptidique et un pont disulfure entre les groupes thiol des résidus de cystéine 4 et 13, qui possède une activité anti-inflammatoire et/ou anti-allergique.

2. Peptide selon la revendication 1, **caractérisé en ce qu'**il a une séquence d'acides aminés choisie dans le groupe consistant en :
Ile₁-Pro₂-Arg₃-Cys₄-Arg₅-Lys₆-Met₇-Pro₈-Gly₉-Val₁₀-Lys₁₁-Met₁₂-Cys₁₃ (Seq. ID No. 1);
Val₁-Glu₂-Gln₃-Cys₄-Thr₅-Ile₆-Ile₇-Gly₈-Asp₉-Glu₁₀-Lys₁₁-Asp₁₂-Cys₁₃ (Seq. ID No. 2);
Val₁-Glu₂-Gln₃-Cys₄-Thr₅-Ile₆-Ile₇-Gly₈-Asp₉-Ala₁₀-Lys₁₁-Asp₁₂-Cys₁₃ (Seq. ID No. 3) ;
Val₁-Gln₂-Gln₃-Cys₄-Ser₅-Glu₆-Ile₇-Ala₈-Gly₉-Ala₁₀-Lys₁₁-Pro₁₂-Cys₁₃ (Seq. ID No. 4) ;
Leu₁-His₂-Arg₃-Cys₄-Asp₅-Lys₆-Ile₇-Ala₈-Asp₉-Ala₁₀-Lys₁₁-Pro₁₂-Cys₁₃ (Seq. ID No. 5) ;
Ile₁-Pro₂-Arg₃-Cys₄-Argₛ-Ala₆-Met₇-Pro₈-Gly₉-Val₁₀-Lys₁₁-Met₁₂-Cys₁₃ (Seq. ID No. 6) ;
Ile₁-Pro₂-Arg₃-Cys₄-Arg₅-Lys₆-Met₇-Pro₈-Gly₉-Val₁₀-Lys₁₁-Met₁₂-Cys₁₃-NH₂ (Seq. ID No. 7) ;
Ac-Ile₁-Pro₂-Arg₃-Cys₄-Arg₅-Lys₆-Met₇-Pro₈-Gly₉-Val₁₀-Lys₁₁-Met₁₂-Cys₁₃ (Seq. ID No. 8) et
Ac-Ile₁-Pro₂-Arg₃-Cys₄-Arg₅-Lys₆-Met₇-Pro₈-Gly₉-Val₁₀-Lys₁₁-Met₁₂-Cys₁₃-NH₂ (Seq. ID No. 9)

3. Peptide selon les revendications 1 ou 2, **caractérisé par le fait que** les acides aminés sont des acides L-aminés.

4. Peptide selon la revendication 2, **caractérisé par le fait que** les résidus de proline sont à titre individuel, en alternance ou conjointement, substitués par des résidus d'hydroxyproline.

5. Peptide selon la revendication 2, **caractérisé par le fait que** les résidus de méthionine sont à titre individuel, en alternance ou conjointement, substitués par des résidus de méthionine oxydée.

6. Peptide selon la revendication 2, **caractérisé par le fait que** les acides L-aminés sont à titre individuel, en alternance ou conjointement, substitués par des acides D-aminés.

7. Peptide selon les revendications 1 ou 2, **caractérisé en ce qu'**il est anti-asthmatique.

8. Peptide selon la revendication 1, **caractérisé en ce qu'**il s'agit d'inhibiteurs de la trypsine.

9. Composition pharmaceutique, **caractérisée en ce qu'**elle comprend un peptide des revendications 1 ou 2, seul ou dans une combinaison contenant au moins deux des peptides, ou leurs sels pharmaceutiquement acceptables, et un ou plusieurs excipients pharmaceutiquement acceptables, le peptide étant présent à une dose efficace pour traiter ou prévenir des troubles inflammatoires et/ou allergiques aigus et/ou chroniques.

10. Composition pharmaceutique selon la revendication 9, **caractérisée en ce qu'**elle est administrée par voie orale, intranasale, intramusculaire ou intraveineuse.

11. Utilisation d'au moins l'un des peptides tels que décrits dans les revendications 1 ou 2, **caractérisée en ce qu'**ils sont utilisés pour la fabrication d'un médicament destiné au traitement ou à la prévention, chez un mammifère, de maladies inflammatoires et/ou allergiques aiguës et/ou chroniques, de l'asthme, de la rhinite, de l'arthrite, de la dermatite atopique, des troubles du système immunitaire, du dysfonctionnement lymphocytaire pendant une réponse immune, des tumeurs, de l'adhésion cellulaire et/ou des maladies parasitaires.

12. Utilisation selon la revendication 11, **caractérisée par le fait que** les peptides sont administrés par voie orale, intranasale, intramusculaire ou intraveineuse.

13. Peptides selon l'une quelconque des revendications 1 à 8, pour utilisation dans le traitement de maladies inflammatoires et/ou allergiques aiguës et/ou chroniques, de l'asthme, de la rhinite, de l'arthrite, de la dermatite atopique, des troubles du système immunitaire, du dysfonctionnement lymphocytaire pendant une réponse immune, des tumeurs, de l'adhésion cellulaire et/ou des maladies parasitaires.


### SEQUENCE LISTING

<110> CRISTALIA PRODUTOS QUIMICOS FARMACEUTICOS LTDA FUNDACAO DE AMPARO A PESQUISA DO ESTADO DE SAO PAULO-FAPESP FERREIRA, MONICA VALDYRCE DOS ANJOS LOPES
<120> ANTI-INFLAMMATORY AND ANTIALLERGIC CYCLIC PEPTIDES
<130> PI0602885-3
<140> PCT/BR2007/
   <141> 2007-07-19
<150> PI0602885-3
   <151> 2006-07-21
<150> 20070090499
   <151> 2007-07-04
<160> 9
<170> PatentIn version 3.3
<210> Sequence ID No.1: (Seq01)
   <211> 13
   <212> PRT
   <213> Artificial
<220>
   <223> Artificial sequence: anti-inflammatory and antiallergic peptides.
<400> 1
   Ile Pro Arg Cys Arg Lys Met Pro Gly Val Lys Met Cys
   1               5                   10
<210> Sequence ID No.2: (Seq02)
   <211> 13
   <212> PRT
   <213> Artificial
<220>
   <223> Artificial sequence: anti-inflammatory and antiallergic peptides.
<400> 2
   Val Glu Gln Cys Thr Ile Ile Gly Asp Glu Lys Asp Cys
   1               5                   10
<210> Sequence ID No.3: (Seq03)
   <211> 13
   <212> PRT
   <213> Artificial
<220>
   <223> Artificial sequence: anti-inflammatory and antiallergic peptides.
<400> 3
   Val Glu Gln Cys Thr Ile Ile Gly Asp Ala Lys Asp Cys
   1               5                   10
<210> Sequence ID No.4: (Seq04)
   <211> 13
   <212> PRT
   <213> Artificial
<220>
   <223> Artificial sequence: anti-inflammatory and antiallergic peptides.
<400> 4
   Val Gln Gln Cys Ser Glu Ile Ala Gly Ala Lys Pro Cys
   1               5                   10
<210> Sequence ID No.5: (Seq05)
   <211> 13
   <212> PRT
   <213> Artificial
<220>
   <223> Artificial sequence: anti-inflammatory and antiallergic peptides.
<400> 5
   Leu His Arg Cys Asp Lys Ile Ala Asp Ala Lys Pro Cys
   1               5                   10
<210> Sequence ID No.6: (Seq06)
   <211> 13
   <212> PRT
   <213> Artificial
<220>
   <223> Artificial sequence: anti-inflammatory and antiallergic peptides.
<400> 6
   Ile Pro Arg Cys Arg Ala Met Pro Gly Val Lys Met Cys
   1               5                   10
<210> Sequence ID No.7: (Seq07)
   <211> 13
   <212> PRT
   <213> Artificial
<220>
   <223> Artificial sequence: anti-inflammatory and antiallergic peptides. NH₂ is a carboxy terminal amide protecting group.
<400> 7
<210> Sequence ID No.8: (Seq08)
   <211> 13
   <212> PRT
   <213> Artificial
<220>
   <223> Artificial sequence: anti-inflammatory and antiallergic peptides. Ac is an acetyl, amino terminal protecting group.
<400> 8
<210> Sequence ID No.9: (Seq09)
   <211> 13
   <212> PRT
   <213> Artificial
<220>
   <223> Artificial sequence: anti-inflammatory and antiallergic peptides. Ac is an acetyl, amino terminal protecting group. NH₂ is a carboxy terminal amide protecting group.
<400> 9